(19) Europäisches Patentamt
European Patent Office
Office européen des brevets

(11) Publication number: 0 006 766
B1

(12) # EUROPEAN PATENT SPECIFICATION

(45) Date of publication of patent specification: 26.01.83

(21) Application number: 79301278.2

(22) Date of filing: 02.07.79

(51) Int. Cl.³: C 07 C 101/42, A 61 K 31/165, A 61 K 31/24, C 07 C 103/28, C 07 C 103/76

(54) Phenethanolamines, their formulations, use and preparation.

(30) Priority: 03.07.78 US 921670

(43) Date of publication of application: 09.01.80 Bulletin 80/1

(45) Publication of the grant of the patent: 26.01.83 Bulletin 83/4

(84) Designated Contracting States: DE GB IT NL SE

(56) References cited: DE - A - 1 643 224

(73) Proprietor: ELI LILLY AND COMPANY
307, East McCarty Street
Indianapolis Indiana 46285 (US)

(72) Inventor: Mills, Jack
7902 Timberhill Drive
Indianapolis, Indiana, 46217 (US)
Inventor: Schmiegel, Klaus Kurt
4507 Staughton Drive
Indianapolis, Indiana 46226 (US)
Inventor: Shaw, Walter Norman
4511 North Broadway
Indianapolis, Indiana 46205 (US)

(74) Representative: Crowther, Terence Roger
European Patent Attorney et al,
Erl Wood Manor
Windlesham Surrey GU20 6PH (GB)

EP 0 006 766 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Phenethanolamines, their formulations, use and preparation

This invention relates to phenethanolamine derivatives, pharmaceutical formulations containing those compounds, methods of preparing the novel compounds and their use in weight control of mammals.

Some of the most extensive efforts in basic research have been in the area of β-phenylethylamine derivatives, many of which are catecholamines. A great deal of study, for example, has been carried out on the naturally occurring catecholamine epinephrine. Epinephrine is a potent sympathomimetic drug and a powerful cardiac stimulant. The use of epinephrine is limited, however, due to its undesirable side effects, which include fear, anxiety, tremor, tenseness, throbbing headache, increased blood pressure, dizziness, respiratory difficulty and palpitation, as well as its short duration of action.

The use of drugs which cause more than one biological effect can be very dangerous. For example, since both bronchodilation and cardiac stimulation are mediated by the broad group of receptors known as β-receptors, a drug acting on such β-receptors not only would effect bronchodilation, but additionally could cause observable effects upon the heart. In fact, some individuals allegedly have died from ventricular fibrillation caused by excessive β-stimulation after using bronchodilation agents; (See Greenburg and Pines, *Br. Med. J. 1,* 563 (1967)).

The cardiac effects of phenethylamine derivatives has long been known — see for instance U.S. Patent Specification No. 3,816,516 which describes the β-agonist properties of certain 1-phenyl-2-alkylaminoalkanol derivatives similar to the compounds of the invention.

The Applicants have now discovered that surprisingly certain *para*-substituted N-phenylpropyl phenethanolamines are effective in causing loss, or preventing formation, of adipose tissue in mammals and that this hitherto unsuspected activity in this type of compound is obtained with minimal associated cardiac effects.

According to the present invention there is provided a compound of the formula (I):

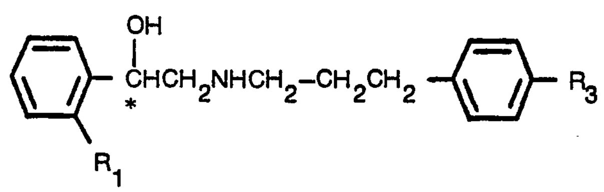

(I)

wherein:

$R_1$ is hydrogen or fluorine;

$R_3$ is aminocarbonyl, methylaminocarbonyl, or $C_1$—$C_2$ alkoxycarbonyl;

or a pharmaceutically-acceptable acid addition salt thereof.

In one aspect of the invention there is provided a pharmaceutical formulation which comprises a compound of formula (I), or a pharmaceutically-acceptable acid addition salt thereof, associated with a pharmaceutically-acceptable carrier therefor.

The invention also provides a compound of formula (I), as defined above, or a pharmaceutically-acceptable acid addition salt thereof for use in removing or preventing the formation of adipose tissue in mammals.

Whilst the compounds provided by this invention are referred to generically as phenethanolamines, they will be systematically named herein as N-substituted-3-phenylpropylamines. For example, a compound having the above formula wherein $R_1$ and $R_2$ both are hydrogen and $R_3$ is aminocarbonyl will be named herein as: N-(2-phenyl-2-hydroxyethyl)-3-(4-aminocarbonylphenyl)propylamine.

The asymmetric carbon atom, namely the carbon atom labeled "C*" in the above formula, preferably has the R-stereochemical configuration as described by Cahn *et al., Experientia,* Vol. XII, pp. 81—124 (1956).

The compounds provided by this invention can be prepared by any of a number of methods involving well known and routinely used chemical processes. A preferred process involves the reaction of a styrene oxide (preferably optically active) with a 3-phenylpropylamine.

According to this aspect of the invention there is provided a method of preparing a compound of formula (I) in which a styrene oxide of formula (II):

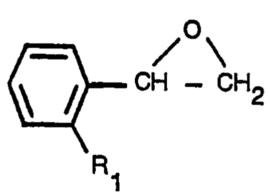

(II)

where $R_1$ is as defined above, is reacted with a propylamine derivative of formula:

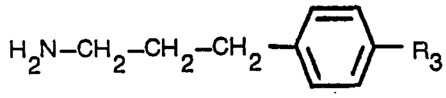

where $R_3$ is as defined above, followed, in the case where the styrene oxide is not optically active, i.e. is not an R-styrene oxide, and it is desired to produce an optically active product, by resolution using conventional methodology.

For example, a styrene oxide, such as R-*ortho*-fluorostyrene oxide, can be reacted with approximately an equimolar quantity of a phenylpropylamine such as 3-(4-methylaminocarbonylphenyl)propylamine to provide the corresponding phenethanolamine of this invention, for instance R—N—[2—(2-fluorophenyl)-2-hydroxyethyl]-3-(4-methylaminocarbonylphenyl)propylamine. Such condensation reactions normally are carried out in an unreactive polar organic solvent such as ethanol, dioxane, toluene or dimethylformamide, and usually at a temperature of from about 20 to about 120°C, preferably from 50 to 110°C. Under such conditions, the condensation generally is substantially complete after about 6 to about 10 hours, and the product phenethanolamine can be isolated by simply removing the reaction solvent, for instance by evaporation under reduced pressure. Further purification of the product thus formed can be accomplished if desired by standard methods including chromatography, crystallization and salt formation.

Another preferred method for preparing the compounds of this invention involves reacting a mandelic acid derivative with a phenylpropylamine to provide the corresponding condensed amide, and then reducing the amide carbonyl group to provide a compound of formula (I). Such a method is ideally suited to the preparation of compounds wherein $R_3$ is an alkoxycarbonyl substituent.

Accordingly in a further aspect of the invention there is provided a method of preparing a compound of formula (I) which comprises reducing a compound of formula (III).

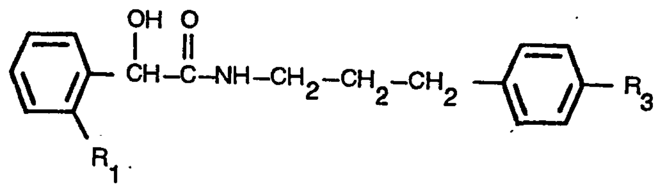

(III)

where $R_1$ and $R_3$ are as defined above, followed in the case where the mandelic acid derivative is not the R-enantiomer, and it is desired to prepare an optically active final product, by resolution using conventional methodology.

The reaction of the mandelic acid derivative with the phenylpropylamine is best accomplished utilizing coupling reagents such as those commonly used in the synthesis of peptides. Such routinely used coupling reagents include carbodiimides such as N,N'-dicyclohexylcarbodiimide (DCC) and N,N'-diisopropylcarbodiimide, as well as N-ethoxycarbonyl-2-ethoxy-1,2-dihydroquinoline (EEDQ). Dicyclohexylcarbodiimide generally is a preferred coupling reagent. A substituted or unsubstituted 1-hydroxybenzotriazole can be employed to accelerate the reaction if desired. The general process for preparing peptides utilizing dicyclohexylcarbodiimide and 1-hydroxybenzotriazole is discussed in detail by Konig and Geiger in *Chem. Ber., 103,* 788—798 (1970). For instance, either 2-phenyl-2-hydroxyacetic acid or 2-(2-fluorophenyl)-2-hydroxyacetic acid can be reacted with approximately an equimolar quantity of a 3-phenylpropylamine in the presence of a substantially equimolar amount of both dicyclohexylcarbodiimide and 1-hydroxybenzotriazole. The coupling reaction is best carried out in an organic solvent such as dimethylformamide, hexamethylphosphorotriamide, acetonitrile or dichloromethane. The reaction normally is carried out at a reduced temperature, for example at a temperature below about 50°C., generally at about 0 to about 10°C. The reaction normally is substantially complete within about two to about twenty hours; however, longer reaction periods appear not to be detrimental to the desired product and can be utilized if desired.

The coupling of an acid and an amine in the presence of dicyclohexylcarbodiimide to form an amide converts the dicyclohexylcarbodiimide to dicyclohexylurea. This latter compound is characteristically quite insoluble in organic solvents and accordingly can be removed from the reaction mixture simply by filtration. Once the dicyclohexylurea is removed from the reaction mixture, the product amide can be isolated by removal of the reaction solvent, for instance by evaporation of the solvent under a reduced pressure. The product thus formed can be dissolved in a water immiscible organic solvent such as ethyl acetate and washed with aqueous sodium bicarbonate to remove any remaining 1-hydroxybenzotriazole. The amide so formed can, if desired, be further purified by routine procedures including crystallization and chromatography.

The amide next is reduced to provide the corresponding N-phenylpropyl phenethanolamine comprehended by this invention. Reduction of such amide can be accomplished by any of a number of routine reduction processes, such as reaction with metal hydride reducing agents. A preferred reduction process comprises reaction of an amide with diborane. In such a reduction reaction, the diborane typically is utilized in an excess amount relative to the amide, such as from about 1 to about 4 molar excess. The reduction generally is carried out in an organic solvent such as tetrahydrofuran, diethyl ether, benzene, dichloromethane, toluene, dioxane, or like solvents. Such reduction reactions typically are complete within about two to twenty hours when carried out at a temperature of from about 0° to about 100°C. Any excess diborane and borane complex remaining in the reaction mixture after the reduction of the amide is complete can be decomposed by the addition to the reaction mixture of an alcohol such as methanol or ethanol and an acid such as hydrochloric acid. The reduced product thus formed can be isolated by simply removing the reaction solvent, for example by evaporation. The product, a phenethanolamine having the above formula, generally exists as a solid and can thus be further purified by crystallization or chromatography. Alternatively, the amine so formed can be converted to an acid addition salt, as will be discussed more fully hereinbelow.

When the above-described processes for preparing a compound of formula (I) are carried out utilizing unresolved starting materials, thereby affording racemic mixtures of N-phenylpropyl phenethanolamines, separation of the diastereomers so formed can be accomplished by conventional techniques known *per se* in the art, if required. However, since the S-isomer is substantially inactive, having no adverse side-effects at the therapeutically-effective dose levels, the racemate is advantageously utilized without resolution. However, it should be emphasized that it is the R-isomer which conveys the biological activity.

The quality of meat, in livestock animals such as pigs, sheep or cattle is improved by administering to those animals a compound of formula (I). This improvement in meat quality may take place in two ways; firstly, adipose tissue already present may be removed and, secondly, formation of adipose tissue may be prevented. The compound of formula (I) will normally be administered to the animal in its feed.

Accordingly, in a further aspect of the invention there is provided animal feed containing a compound of formula (I), or a pharmaceutically-acceptable salt thereof.

Another method of preparing compounds comprehended by this invention comprises reacting a phenethanolamine with an aldehyde or ketone to provide the corresponding Schiff base, followed by reduction of the Schiff base. Such process is illustrated by the following scheme:

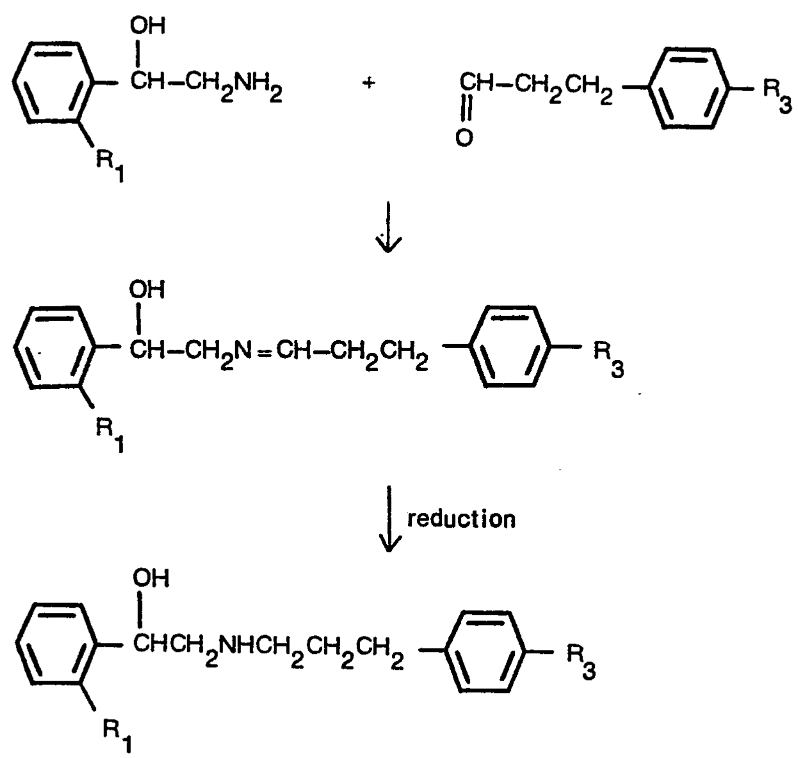


The condensation of the phenethanolamine and the phenethyl aldehyde can be carried out in an organic solvent such as toluene, generally in the presence of an acidic catalyst such as *para*-toluenesulfonic acid. The product from the condensation is an imine, i.e., a Schiff base, which upon reduction provides the corresponding phenethanolamine of the invention. The reduction is typically carried out

with hydrogen or with a metal hydride such as sodium cyanoborohydride.

Still another method for preparing the compounds of this invention comprises reacting a hydroxy protected 2-phenyl-2-hydroxyacetic acid acylating agent with a 3-phenylpropylamine to provide an amide, and then reduction of the amide carbonyl group to provide a compound of this invention. Such process is depicted by the following scheme:

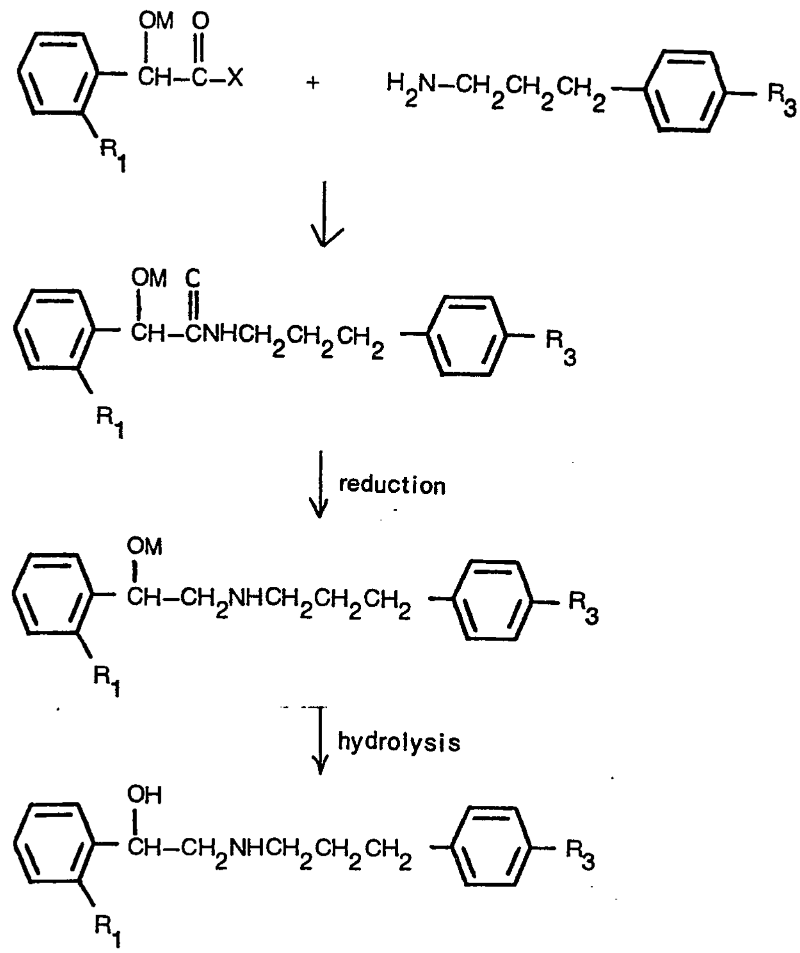


$R_1$ and $R_3$ in the above formulae are as defined above, X is a good leaving group and M is a readily removable hydroxy protecting group. Commonly used hydroxy protecting groups include acyl moieties such as acetyl, chloroacetyl and dichloroacetyl, as well as ether forming groups such as trimethylsilyl and the like. Such readily removable hydroxy protecting groups are more fully described by E. Haslam in *Protective Groups in Organic Chemistry,* J.F.W. McOmie, Ed., Plenum Press, New York, N.Y., 1973, Chapter 3. As noted above, X is defined as a good leaving group and includes halogen groups such as chloride and bromide, as well as acyloxy groups such as acetoxy and dichloroacetoxy. As an example of the preparation of a compound of this invention utilizing a hydroxy protected mandelic acid acylating agent, a compound such as R-2-(2-fluorophenyl)-2-hydroxyacetic acid can be reacted with dichloroacetyl chloride to provide R-2-(2-fluorophenyl)-2-dichloroacetoxyacetic acid. Reaction of this latter compound with a halogenating agent such as thionyl chloride or oxalyl chloride provides the corresponding acid chloride, namely R-2-(2-fluorophenyl)-2-dichloroacetoxyacetyl chloride. Reaction of such acid chloride with a phenylpropylamine provides the corresponding amide. The amide so formed is then reduced, for example by reaction with diborane or the like, and the hydroxy protected group is removed, for instance by hydrolysis, thus providing an optically active compound having the above formula.

A further method for preparing compounds comprehended by this invention comprises reacting a phenacyl halide with a 3-phenylpropylamine. For example, a phenacyl halide such as 2-fluorophenacyl bromide can be reacted with about an equimolar quantity of an amine such as 3-(4-hydroxyphenyl)-propylamine. Such alkylation reactions normally are carried out in a suitable solvent such as ethanol and in the presence of a base such as sodium carbonate or triethylamine. The reaction is complete within about six hours when carried out at about 50°C. and provides a ketone intermediate, for instance N-[2-(2-fluorophenyl)-2-oxoethyl]-3-(4-hydroxyphenyl)propylamine. The ketone thus formed can be isolated as the free amine base or as an acid addition salt. Reduction of such ketone, for example

by reaction with sodium borohydride or the like, affords a compound of this invention generally as a mixture of optical isomers at $\overset{*}{C}$. Such mixture of optical isomers can be separated by standard procedures, or if desired can be utilized directly in the present invention, since the S isomer is essentially devoid of biological activity.

Compounds of the above formula wherein $R_3$ is aminocarbonyl can be preapred either directly by reacting the appropriate phenylpropylamine with a styrene oxide or alternatively can be derived from those compounds wherein $R_3$ is $C_1$—$C_2$ alkoxycarbonyl, i.e. methoxycarbonyl or ethoxycarbonyl. For example, R-mandelic acid can be coupled with 3-(4-methoxycarbonylphenyl)propylamine to provide, after reduction of the amide carbonyl group, the corresponding optically-active amine namely R-N-(2-phenyl-2-hydroxyethyl)-3-(4-methoxycarbonylphenyl)propylamine. This amine can be reacted with hydrazine for about 2 to 40 hours at about 50 to 100°C. to convert the alkoxycarbonyl group to a hydrazino carbonyl group. The latter compound can be converted by hydrogenation to the corresponding aminocarbonyl derivative. Such hydrogenation is accomplished utilizing common catalysts such as Raney nickel.

Compounds of formula (I) wherein $R_3$ is methylaminocarbonyl are preferably prepared by reacting a styrene oxide with a 3-(4-methylaminocarbonylphenyl)propylamine. The required phenylpropylamine starting material can be prepared by simply reacting methylamine with the appropriate benzoyl chloride derivative. For example, 3-(4-chlorocarbonylphenyl)propylaminium chloride can be reacted with methylamine to provide 3-(4-methylaminocarbonylphenyl)propylamine. Reaction of the latter compound with a styrene oxide affords a compound of formula (I).

The compounds provided by this invention are amines and as such are basic in nature. Consequently, they can readily be converted to acid addition salts by reaction with organic or inorganic acids. Accordingly, an additional embodiment of this invention comprises pharmaceutically-acceptable acid addition salts of the N-phenylpropyl phenethanolamines of the above formula. The particular acids utilized to form salts of this invention are not critical, and such salts include those which are prepared by reaction of the amine of this invention with any of a number of common acids such as hydrochloric, hydrobromic, sulfuric, phosphoric, nitric, perchloric, formic, acetic, butyric, citric, maleic, succinic, oxalic, fumaric, lactic, methanesulfonic, p-toluenesulfonic, and related acids. The pharmaceutically acceptable acid addition salts which are formed by reaction of an amine of this invention with an acid such as one of the above-named acids typically exist as highly crystalline solids, and thus lend themselves to ready purification by recrystallization from common solvents such as methanol, ethanol, ethyl acetate and the like. Additionally, such salts are easily formulated for convenient administration, particularly by the oral route, to subjects in need of treatment for obesity. When desired, such acid addition salts are readily converted to the corresponding free amine base by reaction with a suitable basic compound, for instance sodium or potassium hydroxide, sodium carbonate, triethylamine, sodium bicarbonate, and the like.

Obesity is a very serious disease that currently is the subject of a great deal of concern, particularly since no truly effective treatments are known. A full discussion of nutritional diseases and obesity in general is presented by Albrink in *Textbook of Medicine,* 12th Ed., 1969, W. B. Saunders Company, Philadelphia, Pa., pp. 1164—1174 and by Salans in *Current Therapy,* 1977, W. B. Saunders Company, pp. 455—460. Compounds provided by this invention are particularly useful due to their ability to effect an actual reduction in weight when administered to a mature obese animal. Such weight reduction is accomplished without a concomitant reduction in daily food consumption. When the compounds of this invention are administered to immature obese animals, the degree of weight gain is significantly reduced compared to that observed in young obese animals not receiving a compound of the invention.

The anti-obesity activity of the compounds of this invention has been demonstrated in a number of biological tests utilizing mice, rats and dogs. One of the major actions of the claimed compounds on a biological system appears to be the mobilization of fatty acids from adipose tissue stores.

In addition, the compounds of this invention cause an actual reduction in body weight in mature obese animals without causing a decrease in food consumption, i.e., without causing appetite suppression.

As already pointed out, the unique physiological effect of the compounds of this invention is their potent anti-obesity activity and their physiologically tolerable degree of cardiovascular activity. This unique and unexpected separation of biological activity is achieved by selecting the proper chemical substitution pattern in the phenethanolamines of the invention.

Because of the surprisingly low inotropic activity of the compounds of this invention, they can be administered to an animal at doses large enough to effect release of free fatty acids from adipose stores without causing a substantial increase in the pumping force of the heart. Such unique biological spectrum of the compounds of this invention renders them particularly useful in the control of weight in obese animals. As used herein, "weight control in obese animals" refers to the ability of the compounds of this invention to effect an actual weight reduction when administered to a mature obese animal, whereas such compounds are effective in the prevention of excessive weight gain when administered to immature obese animals. The terms "mature" and "immature" are used herein to refer to the

generally accepted definitions of age and growth patterns. "Obesity" is an art recognized term and is used as such herein.

While the anti-obesity effective dose of a compound of this invention employed in the control of obesity will vary depending on the particular drug employed and the severity of the condition being treated, the usual dosage of a compound of this invention will be from about 1.0 to about 25 mg. per kilogram of animal body weight. The compounds of the invention preferably will be administered orally at a dosage of from about 1 to about 5 mg./kg., generally given in individual doses from one to four times per day. When desired, the drug can be administered orally in the form of a tablet or capsule, or alternatively in sustained release form. A compound defined by the above general formula is administered to a mature obese animal to effect an actual reduction in weight without diminishing the daily food consumption. The drug will be administered daily, at increasing dosage levels if desired, until the desired weight reduction is effected. The compounds of the invention can be administered to an immature obese animal to effect a reduction in weight gain without a diminished daily food consumption. Once the immature obese animal reaches maturity, a reduction of weight will be effected until a substantially normal weight is achieved.

The compounds comprehended by this invention can be formulated by normal procedures for conventient administration by any of a number of routes. It is preferred that the compounds be formulated for oral administration. Pharmaceutical formulations which are useful in weight control in obese animals are provided in a further embodiment of this invention.

Such pharmaceutical compositions can contain, as active ingredient, one or more of the compounds provided by this invention, in combination if desired with any of the inactive isomers as hereinbefore indicated, in addition to any of a number of pharmaceutically acceptable carriers or diluents. Typical carriers and diluents commonly used include gelatin, starch, dextrose, sucrose, lactose, cellulose derivatives, stearates, polyvinylpyrrolidine, glycerine, ethyl lactate, sorbitol mannitol, and the like. A suitable pharmaceutical composition can additionally include any of a number of common preserving agents, stabilizers, antioxidants, taste correctors, and the like. Examples of such additives include ascorbic acid, sorbic acid, various esters of p-hydroxybenzoic acid, and the like.

Typical pharmaceutical compositions useful in the treatment of obesity according to this invention will generally include from about 1 to about 50 percent by weight of a compound of this invention as active ingredient. The remainder of said pharmaceutical composition will comprise suitable carriers and diluents.

A pharmaceutical composition containing as active ingredient at least one of the compounds of this invention can be molded into tablets, encapsulated into empty gelatin capsules, or made into a solution or suspension. Such pharmaceutical compositions can be administered to an obese subject in need of treatment by any of a number of routes, including the oral and parenteral routes. A preferred formulation, for example, comprises about 500 mg. of N-(2-phenyl-2-hydroxyethyl)-3-(4-aminocarbonylphenyl)propylaminium chloride admixed with any of a number of suitable carriers and molded into a tablet for oral administation to an obese human subject at the rate of from 1 to about 4 tablets per day for effective weight control.

In an effort to more fully illustrate particular aspects of this invention, the following Preparations illustrate how starting materials useful in the invention may be prepared whereas the following Examples illustrate the preparation of final products of formula (I) and their use in pharmaceutical formulations. The Examples are representative only and should not be construed as limiting in any respect. In the Preparations and Examples the optical rotations were measured at room temperature and at a concentration of 9.3 mg per ml of solvent.

## Preparation 1

Preparation of R-styrene oxide

To a cold stirred solution of 300 g. of R-mandelic acid in 2000 ml. of tetrahydrofuran was added dropwise over two hours 3000 ml. of a 1 molar solution of diborane in tetrahydrofuran. The reaction mixture was stirred for twelve hours at 25°C. following complete addition, and then was cooled to 0°C. in an ice water bath. To the cold solution was added 600 ml. of methanol dropwise over thirty minutes, and the reaction mixture was then stirred for another three hours. Removal of the solvent by evaporation under reduced pressure provided 260 g. of the product as a solid. The solid was crystallized from diethyl ether to provide R-2-phenyl-2-hydroxyethanol.

A solution of 256.7 g. of R-2-phenyl-2-hydroxyethanol in 1000 ml. of toluene containing 50 ml. of pyridine was stirred at 0°C. while a solution of 372.1 g. of *p*-toluenesulfonyl chloride in 400 ml. of toluene was added dropwise over two hours. The reaction mixture was stirred at 0°C. for fortyeight hours and then filtered to remove the precipitated pyridine hydrochloride. The filtrate was concentrated to dryness by evaporation under reduced pressure to provide the product as an oil. The oil was dissolved in fresh toluene, washed with dilute hydrochloric acid and with water, and dried. Evaporation of the solvent under reduced pressure and crystallization of the product from diethyl ether and hexane afforded 435 g. of R-2-phenyl-2-hydroxy-1-*p*-toluenesulfonyloxyethane. The product thus formed was dissolved in 1000 ml. of dimethyl sulfoxide containing 418 ml. of 5 N sodium hydroxide. The alkaline solution was stirred at 0°C. for twelve hours, and then was poured into ice water. The product was

extracted into 50% diethyl etherpentane, and the organic layer was separated, washed with water and dried. Removal of the solvent by evaporation afforded, after distillation, 165 g. of R-styrene oxide. B.P. 55—56°C. 79.99 N/m² 0.6 torr. $[\alpha]_D$ −23.7° (chloroform).

Preparation 2

Preparation of R-*ortho*-fluorostyrene oxide

One hundred three grams of dl-*o*-fluoromandelic acid was resolved by reaction with d(+)-$\alpha$-methylbenzylamine to form the salt, and crystallization of the salt from ethanol and ethyl acetate to provide optically active $\alpha$ - methylbenzylaminium 2 - (2 - fluorophenyl - 2 - hydroxy)acetate, M.P. 155—157°C. $[\alpha]_D$ −43.1° (MeOH). The salt thus formed was hydrolyzed to the free acid to provide R-*ortho*-fluoromandelic acid, M.P. 88—90°C. $[\alpha]_D$ −138° (MeOH). The acid was then converted to R-*ortho*-fluorostyrene oxide by the procedure of Preparation 1, B.P. 58—61°C. 399.9 N/m² (3 torr), $[\alpha]_D$ −32.7° (chloroform).

These optically starting materials may be used to prepare optically-active compounds of formula (I).

Preparation 3

3-(4-Methoxycarbonylphenyl)propylamine

The oil from 55 g. of a 50 percent dispersion of sodium hydride in mineral oil was removed by several washings with toluene under an atmosphere of nitrogen. The sodium hydride was covered with 500 ml. of dimethylformamide and 500 ml. of toluene. The mixture was stirred and cooled in an ice bath. 339 g. of ethyl cyanoacetate were slowly added whilst the temperature was maintained at 25°C. After the addition, a solution of methyl $\alpha$-bromotoluate in 500 ml. of toluene was added slowly. The reaction mixture was heated to reflux for four hour, poured onto an ice-hydrochloric acid mixture and extracted with ether. The ether extracts were washed with water and evaporated under reduced pressure to remove the solvent and excess ethyl cyanoacetate. There remained 218 g. of oily ethyl 3-(4-methoxycarbonylphenyl)-2-cyanopropionate.

A mixture of 26.1 g. of this cyanodiester, 75 ml. of dimethylsulfoxide, 6.4 g. of sodium chloride and 5.4 g. of water was heated at 150°C. until no more carbon dioxide was evolved. The mixture was stripped to dryness, diluted with water and extracted with ethyl acetate. The organic extracts were washed with water, dried; and evaporated. The residue was distilled to yield 6.4 g. of an oil, b.p. 100—120°C. (0.05 mm.), which solidified on standing.

Analysis calc. for $C_{11}H_{11}NO_2$

| | | | |
|---|---|---|---|
| Theory: | C, 69.83; | H, 5.86; | N, 7.40. |
| Found: | C, 69.57; | H, 5.69; | N, 7.62. |

The solidified distillate was hydrogenated in 90 ml. of methanol containing 3.5 ml. of concentrated hydrochloric acid and 1 g. of platinum oxide. The catalyst was removed by filtration and the solvent was evaporated. Recrystallization of the solid from methanol gave 7.4 g. of 3-(4-methoxycarbonylphenyl)propylaminum chloride, m.p. 163—168°C.

Treatment of the hydrochloride salt with sodium hydroxide afforded the title compound as free base which was used immediately in the process of Example 1.

Example 1

dl-N-(2-Phenyl-2-hydroxyethyl)-3-(4-methoxycarbonylphenyl)propylamine

A solution of 7.0 g. of 3-(4-methoxycarbonylphenyl)propylamine in 75 ml. of ethanol containing 4.8 g. of dl-styrene oxide was heated to reflux and stirred for seven hours. The reaction mixture was cooled to room temperature and the solvent was removed by evaporation under reduced pressure to provide the product as a solid residue. The residue was crystallized from diethyl ether to afford 1.7 g. of dl-N-(2-phenyl-2-hydroxyethyl)-3-(4-methoxycarbonylphenyl)propylamine. M.P. 109—112°C. The amine so formed was converted to the hydrochloride salt by reaction with hydrogen chloride in methanol to afford 1.3 g. of dl-N-(2-phenyl-2-hydroxyethyl)-3-(4-methoxycarbonylphenyl)propylaminium chloride. M.P. 179—181°C.

Analysis calc. for $C_{19}H_{24}NO_3Cl$

| | | | |
|---|---|---|---|
| Theory: | C, 65.23; | H, 6.91; | N, 4.00. |
| Found: | C, 65.49; | H, 6.79; | N, 3.97. |

The mixture of optical isomers thus formed can be used as a mixture since the S-isomer is substantially devoid of biological activity. If desired, however, the racemic mixture can be resolved by standard procedures.

Example 2

dl-N-(2-Phenyl-2-hydroxyethyl)-3-(4-aminocarbonylphenyl)propylamine

A solution of 3.4 g. of dl-N-(2-phenyl-2-hydroxyethyl)-3-(4-methoxycarbonylphenyl)propylamine

in 200 ml. of ethanol containing 50 ml. of hydrazine was heated to reflux and stirred for twelve hours. The reaction mixture was cooled to room temperature and concentrated to dryness by evaporation of tha solvent. The residue was crystallized from ethanol to give 3.0 g. of dl-N-(2-phenyl-2-hydroxyethyl)-3-(4-hydrazinocarbonylphenyl)propylamine. M.P. 143—145°C.

Analysis calc. for $C_{18}H_{23}N_3O_2$

| | | | |
|---|---|---|---|
| Theory: | C, 68.98; | H, 7.40; | N, 13.41. |
| Found: | C, 69.25; | H, 7.45; | N, 13.55. |

A solution of 3.0 g. of dl-N-(2-phenyl-2-hydroxyethyl)-3-(4-hydrazinocarbonylphenyl)propylamine in 500 ml. of ethanol was stirred at 25°C. for seventy-two hours under hydrogen in a Brown hydrogenerator and in the presence of four teaspoons full of Raney nickel. Additional catalyst was added to the reaction mixture and stirring was continued for twelve hours. The reaction mixture then was filtered and the solvent was removed from the filtrate by evaporation under reduced pressure to provide a solid residue. The residue was dissolved in ethyl acetate, washed with dilute ammonium hydroxide, with water and dried. Evaporation of the solvent under reduced pressure provided a solid which next was crystallized from methanol to provide 600 mg. of dl-N-(2-phenyl-2-hydroxyethyl)-3-(4-aminocarbonylphenyl)propylamine. M.P. 138—140°C. The amine so formed was converted to the hydrochloride salt by reaction with hydrogen chloride in methanol to provide 600 mg. of dl-N-(2-phenyl-2-hydroxyethyl)-3-(4-aminocarbonylphenyl)propylamine. M.P. 138—140°C. The amine so formed was converted to the hydrochloride salt by reaction with hydrogen chloride in methanol to provide 600 mg. of dl-N-(2-phenyl-2-hydroxyethyl)-3-(4-aminocarbonylphenyl)propylaminium chloride. M.P. 230—232°C.

Analysis calc. for $C_{18}H_{23}N_2O_2Cl$

| | | | |
|---|---|---|---|
| Theory: | C, 64.57; | H, 6.92; | N, 8.37. |
| Found: | C, 64.64; | H, 6.55; | N, 8.51. |

As described in Example 1, the racemic mixture thus formed can be utilized as such or can be separated by standard procedures.

Example 3

Preparation of 250 mg. Tablets

| | |
|---|---|
| N-(2-phenyl-2-hydroxyethyl)-3-(4-aminocarbonylphenyl)-propylaminium chloride | 250 mg. |
| Lactose | 200 mg. |
| Corn Starch | 300 mg. |
| Corn Starch Paste | 50 mg. |
| Calcium Stearate | 5 mg. |
| Dicalcium Phosphate | 45 mg. |

The active drug, corn starch, lactose, and dicalcium phosphate are uniformly blended. The corn starch paste is prepared as a 10 percent aqueous paste and is blended into the mixture to uniformity. The mixture is blended with the calcium stearate and then compressed into tablets. Such tablets can be administered to an obese subject at the rate of 1 to about 4 tablets per day or as needed.

Example 4

Preparation for suppositories

| | |
|---|---|
| N-(2-phenyl-2-hydroxyethyl)-3-(4-methoxycarbonyl)-propylaminium chloride | 500 mg. |
| Theobroma oil | 1500 mg. |

The above ingredients are blended to uniformity at a temperature of about 60°C. and then permitted to cool in a tapered mold. Each suppository will weigh about 2 grams and can be

administered to a mature obese subject from 1 to 2 times each day for the reduction of weight, or to an immature obese subject for the control of weight gain.

Example 5

Preparation for Oral Suspension

| | |
|---|---|
| N-[2-(2-fluorophenyl)-2-hydroxyethyl]-3-(4-methoxycarbonylphenyl)propylaminium acetate | 5000 mg. |
| Sorbitol solution (70% N.F.) | 40 mg. |
| Sodium benzoate | 150 mg. |
| Lactose | 10 mg. |
| Cherry flavor | 50 mg. |
| Distilled water q.s. ad | 100 ml. |

The sorbitol solution is added to 40 ml. of distilled water and the N-[2-(2-fluorophenyl)-2-hydroxyethyl]-3-(4-methoxycarbonylphenyl)-propylaminium acetate is dissolved therein. The lactose, sodium benzoate and flavoring are added and dissolved. The volume of the solution is adjusted to 100 ml. with distilled water. Each ml. of syrup contains 50 mg. of active drug. A mature obese mammal will be administered about 5 to about 20 ml. of syrup each day for the effective loss of weight.

Examples 6 and 7

As stated previously, the compounds of the invention are particularly useful in improving the meat of pigs and accordingly, typical rations suitable for growing or finishing swine are listed below:

SWINE GROWER RATION

| Ingredient | Lbs./ton |
|---|---|
| Corn, yellow, ground | 1464 |
| Alfalfa meal, dehydrated, 17% | 50 |
| Soybean oil meal, solvent extracted dehulled, 50% | 246 |
| Meat scraps, 55% | 50 |
| Fish meal with solubles | 50 |
| Distillers dried solubles (corn) | 50 |
| Animal fat | 40 |
| Calcium carbonate | 14 |
| Dicalcium phosphate, feed grade | 10 |
| Salt (NaCl) | 10 |
| Trace mineral premix | 2 |
| Swine vitamin premix | 10 |
| Methionine hydroxy analogue, 90% | 4 |
| Total | 2000 |

A typical swine finisher ration contains the following ingredients.

SWINE FINISHER

| Ingredient | Percent | Lbs./ton |
|---|---|---|
| Corn, yellow, ground | 78.25 | 1565 |
| Alfalfa meal, dehydrated, 17% | 2.50 | 50 |
| Soybean oil meal, solvent extracted, dehulled, 50% | 10.50 | 210 |
| Meat scraps, 55% | 2.50 | 50 |
| Distillers dried solubles (corn) | 2.50 | 50 |
| Animal fat | 2.00 | 40 |
| Dicalcium phosphate, feed grade | 0.50 | 10 |
| Calcium carbonate | 0.40 | 8 |
| Salt (NaCl) | 0.50 | 10 |
| Trace mineral premix | 0.10 | 2 |
| Swine vitamin premix | 0.25 | 5 |
| Total | 100.00 | 2000 |

The compounds of the invention are incorporated in these rations in sufficient amount that the pig receives from 5 to 250 mg/kg of feed.

**Claims**

1. A compound of formula (I)

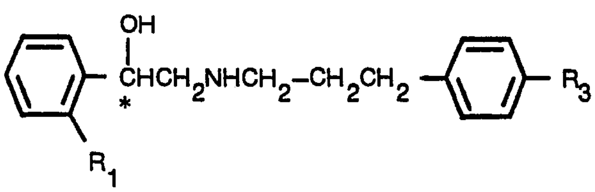

(I)

wherein:

$R_1$ is hydrogen or fluorine;

$R_3$ is aminocarbonyl, methylaminocarbonyl or $C_1$—$C_2$ alkoxycarbonyl; or a pharmaceutically-acceptable acid addition salt thereof.

2. A compound of formula (I) in which the C* carbon atom has the R absolute stereochemical configuration.

3. A compound according to Claim 1 or 2, wherein $R_1$ is hydrogen.

4. A compound according to Claim 1, 2 or 3, wherein $R_3$ is aminocarbonyl.

5. A compound according to Claim 1, 2 or 3, wherein $R_3$ is methoxycarbonyl.

6. N-(2-Phenyl-2-hydroxyethyl)-3-(4-methoxycarbonylphenyl)propylamine.

7. N-(2-Phenyl-2-hydroxyethyl)-3-(4-aminocarbonylphenyl)propylamine.

8. A pharmaceutical formulation comprising a compound of formula (I) as claimed in any one of Claims 1 to 7, or a pharmaceutically-acceptable acid addition salt thereof, associated with a pharmaceutically-acceptable carrier therefor.

9. A compound of formula (I) as claimed in any one of Claims 1 to 7 or a pharmaceutically-acceptable acid addition salt thereof, for use in removing or preventing the formation of adipose tissue in mammals.

10. A process for preparing a compound of formula (I) as claimed in any one of Claims 1 to 7, which process comprises:

(a) reacting a styrene oxide of formula (II):

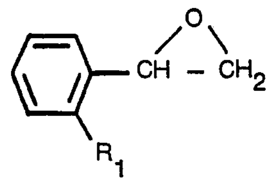 (II)

wherein $R_1$ is as defined in Claim 1, with a propylamine derivative of formula:

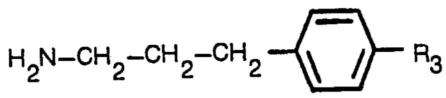

wherein $R_3$ is as defined in Claim 1;

(b) reducing a compound of formula (III):

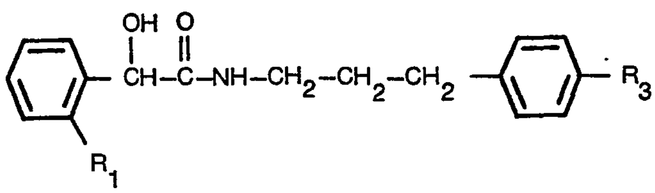 (III)

where $R_1$ and $R_3$ are as defined above;

(c) reducing an imine of formula:

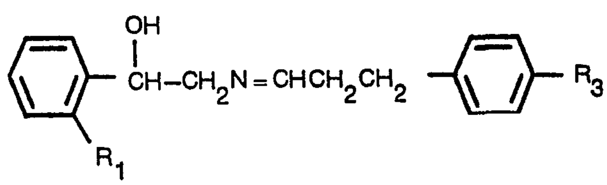

where $R_1$ and $R_3$ are as defined above; or

(d) deprotecting a hydroxyl protected compound of formula:

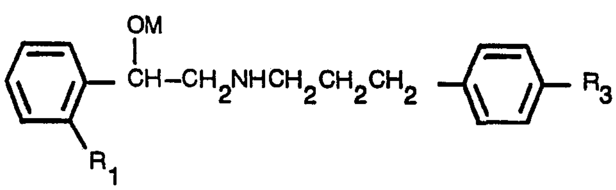

wherein $R_1$ and $R_3$ are as defined above, and M is a readily removable hydroxy protecting group; and

(e) in the case where the product of reaction (a), (b), (c) or (d) is a racemate and it is desired to produce the R-isomer, resolving the racemic product.

11. A process according to claim 10, wherein the racemate of formula (I) is produced by step (a) or (b).

12. Animal feed comprising a compound of formula (I) as claimed in any one of claims 1 to 7 or a pharmaceutically-acceptable acid addition salt thereof.

**Revendications**

1. Composé répondant à la formule (I):

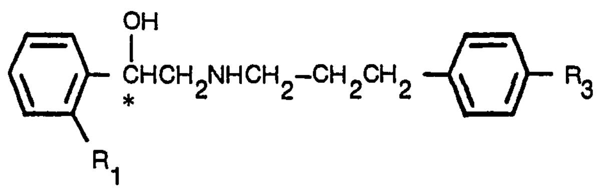

(I)

dans laquelle

$R_1$ représente un atome d'hydrogène ou un atome de fluor;

$R_3$ représente un groupe aminocarbonyle, un groupe méthylaminocarbonyle ou un groupe alcoxy-(en $C_1$—$C_2$)carbonyle; ou

un sel d'addition d'acide pharmaceutiquement acceptable de ce composé.

2. Composé de formule (I) dans laquelle l'atome de carbone C* a la configuration stéréochimique absolue R.

3. Composé suivant la revendication 1 ou 2, caractérisé en ce que $R_1$ représente un atome d'hydrogène.

4. Composé suivant la revendication 1, 2 ou 3, caractérisé en ce que $R_3$ représente un groupe aminocarbonyle.

5. Composé suivant la revendication 1, 2 ou 3, caractérisé en ce que $R_3$ est un groupe méthoxycarbonyle.

6. La N-(2-phényl-2-hydroxyéthyl)-3-(4-méthoxycarbonylphényl)propylamine.

7. La N-(2-phényl-2-hydroxyéthyl)-3-(4-aminocarbonylphényl)propylamine.

8. Formulation pharmaceutique comprenant un composé de formule (I) suivant l'une quelconque des revendications 1 à 7, ou un sel d'addition d'acide pharmaceutiquement acceptable de ce composé, en association avec un support pharmaceutiquement acceptable pour ce composé ou ce sel.

9. Composé de formule (I) suivant l'une quelconque des revendications 1 à 7 ou un sel d'addition d'acide pharmaceutiquement acceptable de ce composé en vue de l'utiliser pour éliminer ou prévenir la formation de tissus adipeux chez les mammifères.

10. Procédé de préparation d'un composé de formule (I) suivant l'une quelconque des revendications 1 à 7, caractérisé en ce qu'il comprend les étapes qui consistent à:

(a) faire réagir un oxyde de styrène de formule (II):

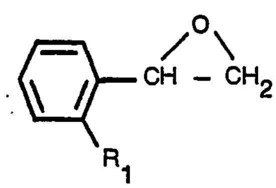

(II)

dans laquelle $R_1$ a la signification définie à la revendication 1, avec un dérivé de propylamine de formule:

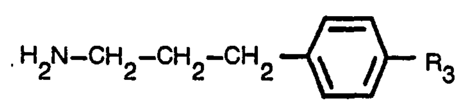

dans laquelle $R_3$ a la signification définie dans la revendication 1;

(b) réduire un composé de formule (III):

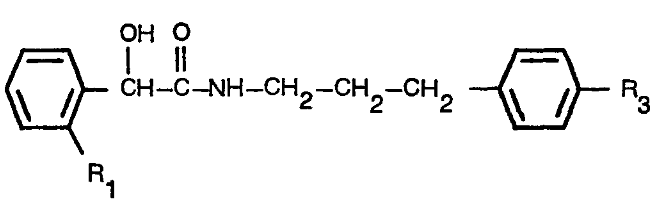

(III)

dans laquelle $R_1$ et $R_3$ ont les significations définies ci-dessus;

(c) réduire une imine de formule:

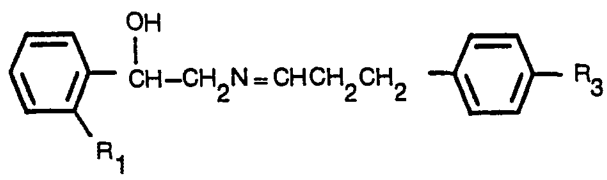

dans laquelle $R_1$ et $R_3$ ont les significations définies ci-dessus; ou

(d) déprotéger un composé protégé sur le groupe hydroxy et répondant à la formule:

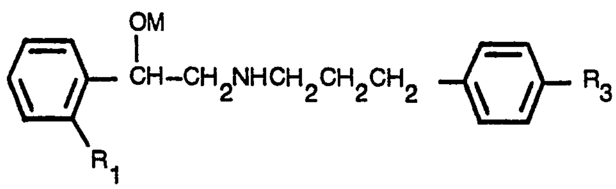

dans laquelle $R_1$ et $R_3$ ont les significations définies ci-dessus et M représente un groupe protecteur du groupe hydroxy pouvant être éliminé aisément; et

(e) lorsque le produit de la réaction (a), (b), (c) ou (d) est un racémate et que l'on désire obtenir l'isomère R, dédoubler le produit racémique.

11. Procédé suivant la revendication 10, caractérisé en ce que le racémate de formule (I) est obtenu par l'étape (a) ou (b).

12. Aliment pour animaux comprenant un composé de formule (I) suivant l'une quelconque des revendications 1 à 7 ou un sel d'addition d'acide pharmaceutiquement acceptable de ce composé.

**Patentansprüche**

1. Phenethanolamine der Formel I

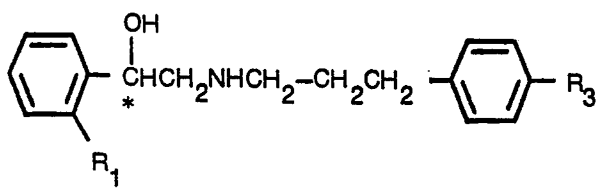

(I)

worin

$R_1$ Wasserstoff oder Fluor ist und

$R_3$ Aminocarbonyl, Methylaminocarbonyl oder $C_1$—$C_2$-Alkoxycarbonyl bedeutet,

und die pharmakologisch unbedenklichen Säureadditionssalze hiervon.

2. Verbindung der Formel (I), worin das Kohlenstoffatom C die absolute stereochemische Konfiguration R hat.

3. Verbindung nach Anspruch 1 oder 2, dadurch gekennzeichnet, daß $R_1$ Wasserstoff ist.

4. Verbindung nach Anspruch 1, 2 oder 3, dadurch gekennzeichnet, daß $R_3$ Aminocarbonyl bedeutet.

5. Verbindung nach Anspruch 1, 2 oder 3, dadurch gekennzeichnet, daß $R_3$ Methoxycarbonyl ist.

6. N-(2-Phenyl-2-hydroxyethyl)-3-(4-methoxycarbonylphenyl)propylamin.

7. N-(2-Phenyl-2-hydroxyethyl)-3-(4-aminocarbonylphenyl)propylamin.

8. Pharmazeutische Formulierung aus einer Verbindung der Formel (I) gemäß einem der Ansprüche 1 bis 7 oder einem pharmakolgisch unbedenklichen Säureadditionssalz hiervon in Verbindung mit einem pharmazeutisch unbedenklichen Träger hierfür.

9. Verwendung einer Verbindung der Formel (I) nach einem der Ansprüche 1 bis 7 oder eines pharmakologisch unbedenklichen Säureadditionssalzes hiervon zur Entfernung oder Verhinderung der Bildung von Adipogewebe bei Säugetieren.

10. Verfahren zur Herstellung einer Verbindung der Formel (I) nach einem der Ansprüche 1 bis 7, dadurch gekennzeichnet, daß man

(a) ein Styroloxid der Formel II

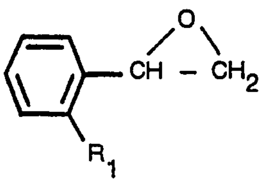

(II)

worin $R_1$ die in Anspruch 1 angegebene Bedeutung hat, mit einem Propylaminderivat der Formel

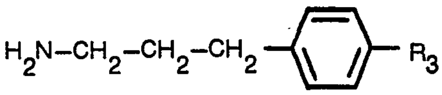

worin $R_3$ die in Anspruch 1 genannte Bedeutung besitzt, umsetzt oder

(b) eine Verbindung der Formel III

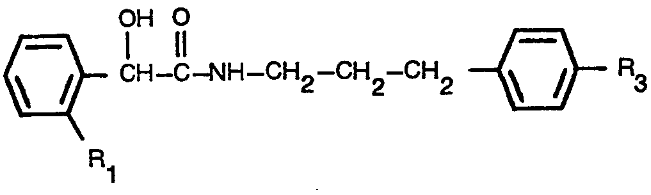

(III)

worin $R_1$ und $R_3$ die oben angegebenen Bedeutungen haben, reduziert oder

(c) ein Imin der Formel

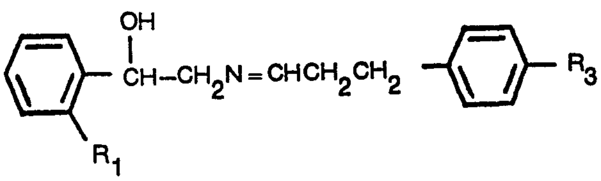

worin $R_1$ und $R_3$ die oben angegebenen Bedeutungen besitzen, reduziert oder

(d) eine hydroxylgeschützte Verbindung der Formel

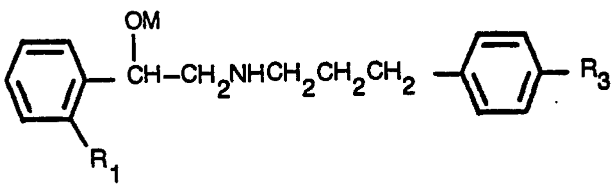

worin $R_1$ und $R_3$ die oben angegebenen Bedeutungen besitzen und M für eine leicht abspaltbare Hydroxylschutzgruppe steht, von der Schutzgruppe befreit, und

(e) falls das Produkt der Umsetzung (a), (b), (c) oder (d) ein Racemat ist und man das R-Isomer bilden möche, das racemische Produkt auftrennt.

11. Verfahren nach Anspruch 10, dadurch gekennzeichnet, daß man das Racemat der Formel (I) nach den Stufen (a) oder (b) herstellt.

12. Tierfutter, gekennzeichnet durch einen Gehalt einer Verbindung der Formel (I) gemäß einem der Ansprüche 1 bis 7 oder eines pharmakologisch unbedenklichen Säureadditionssalzes hiervon.